# EUROPÄISCHE PATENTSCHRIFT

(11) **EP 3 829 550 B1**
(45) Veröffentlichungstag und Bekanntmachung des Hinweises auf die Patenterteilung: **20.12.2023**
(21) Anmeldenummer: 19749316.6
(22) Anmeldetag: 31.07.2019
(51) Int. Cl.: A61K 9/70, A61K 31/00, A61P 43/00, A61P 1/08, A61K 31/46

(54) **TRANSDERMALES THERAPEUTISCHES SYSTEM ZUR ABGABE VON SCOPOLAMIN OHNE MEMBRAN**
TRANSDERMAL THERAPEUTIC SYSTEM FOR DISPENSING SCOPOLAMINE WITHOUT A MEMBRANE
SYSTEME THÉRAPEUTIQUE TRANSDERMIQUE POUR L'ADMINISTRATION DE SCOPOLAMINE

(30) Priorität: 31.07.2018 DE 102018118507
(43) Veröffentlichungstag der Anmeldung: 09.06.2021
(73) Patentinhaber: LTS Lohmann Therapie-Systeme AG, 56626 Andernach (DE)
(72) Erfinder: WIEDERBERG, Sandra, 06268 Steigra (DE); HOFFMANN, Gerd, 56566 Neuwied (DE); MÜLLER, Walter, 56626 Adernach (DE)
(74) Vertreter: Meissner Bolte Partnerschaft mbB
(86) Internationale Anmeldenummer: PCT/EP2019/070667
(87) Internationale Veröffentlichungsnummer: WO 2020/025695

(56) Entgegenhaltungen:
- EP-A2- 0 356 382
- DE-A1- 19 958 554

## Beschreibung

Die vorliegende Erfindung betrifft ein transdermales therapeutisches System ohne freisetzungsbestimmende Membran zur Abgabe von Scopolamin, beruhend auf einem Mehrschichtsystem mit einer Hautkontaktschicht, einer wirkstoffhaltigen Reservoirschicht und einer wirkstoffundurchlässigen Rückschicht sowie gegebenenfalls einer von der Hautkontaktschicht abziehbaren Schutzschicht. Die vorliegende Erfindung betrifft ebenfalls ein Verfahren zur Herstellung von solchen transdermalen therapeutischen Systemen sowie entsprechende Systeme zur Verwendung in der Behandlung von Bewegungskrankheit und/oder postoperativer Übelkeit.

### Stand der Technik

Scopolamin ist ein bekannter Wirkstoff, der sich mit Hilfe eines Pflasters für transdermale Applikation mit systemischer Wirkung eignet. Es handelt sich dabei um ein sogenanntes Antiemetikum, das bevorzugt zur Vermeidung von Übelkeit und Erbrechen, beispielsweise als Folge von wiederholten passiven Veränderungen des Gleichgewichts auf Reisen, eingesetzt wird. Der therapeutische Vorteil einer transdermalen Verabreichung besteht darin, dass die Wirkstoffzufuhr langsam und kontinuierlich unter Steuerung durch das transdermale System erfolgt, wodurch es möglich ist, das vergleichsweise enge therapeutische Fenster für Scopolamin zuverlässig zu treffen und einen therapeutisch effektiven Plasmaspiegel einzustellen, ohne dass die mit einer Überdosierung eintretenden Nebenwirkungen, wie zum Beispiel Mundtrockenheit, Übelkeit und Blendempfindlichkeit der Augen, eintreten.

Auf dem Markt werden transdermale Verabreichungformen von Scopolamin zum Beispiel unter dem Handelsnamen Transderm Scop^{®} in den USA vertrieben. Dieses Pflaster, das im U.S.-Patent 4,904,475 A beschrieben ist, besteht aus vier Schichten, einer Trägerfolie, einem Arzneimittelreservoir, einer die Geschwindigkeit der Wirkstofffreisetzung kontrollierenden Membran und einem mit Wirkstoff beladenen Kontaktkleber. Darüber hinaus ist in Europa ein Membranpflaster unter dem Handelsnamen Scopoderm TTS^{®} erhältlich, das eine die Geschwindigkeit der Freisetzung kontrollierende Membran auf Basis von mikroporösem Polypropylen aufweist. Ein weiteres transdermales Scopolaminpflasterproduct wurde kürzlich von der Firma Aveva/Perrigo vorgestellt. Dieses Pflaster besteht aus einer Rückschicht, einer darauf aufgebrachten Reservoirschicht, einer Ethylen-Vinylacetat Copolymermembran, einer klebenden Hautkontaktschicht und einer abziehbaren Schutzschicht.

Der verhältnismäßig komplizierte Aufbau dieser Freisetzungssysteme führt zu erhöhten Herstellungskosten, da der Herstellungsprozess verhältnismäßig komplex ist. Insgesamt ist zudem für die Herstellung entsprechender Applikationssysteme relativ viel Zeit erforderlich. Ein weiterer Nachteil des als Transderm Scop^{®} TTS bekannten Produkts ist der kalte Fluss, der zur Verunreinigung der Kleidung oder Hände mit der "*toxischen*" Substanz während des Anbringens und/oder Tragens des TTS führt. Zudem muss, um den Wirkstoff gerade zu Beginn der Applikationsdauer in ausreichenden Mengen zur Verfügung stellen zu können, das System thermodynamisch übersättigt sein, was während der Lagerung zu nachteiliger Kristallbildung führen kann.

Die im Markt befindlichen Systeme sind auch vollflächig mit Wirkstoff beladen, so dass es bei der Applikation des transdermalen therapeutischen Systems auf die Haut zu einer Kreuzkontamination (Kontakt von Händen mit Wirkstoff u. anschließendem Kontakt der Hände mit anderen Gegenständen/Körperbereichen) kommen kann. Bei weiterem Kontakt der Hände z.B. mit den Augen kann es aufgrund der Wirkstoffeigenschaften zu einer unerwünschten Pupillenerweiterung kommen.

In der EP 1 011 674 A1 wird ein transdermales therapeutisches System zur Verabreichung von Scopolamin beschrieben, das, wie das Produkt Transderm Scop^{®}, eine die Abgabegeschwindigkeit des Wirkstoffs kontrollierende Membran enthält, die aus einem Ethylenvinylacetatcopolymer besteht. In diesem System wird ein Amin-resistentes Silikonpolymer als Matrix für den Wirkstoff genutzt.

Die deutsche Patentanmeldung DE 198 25 499 A1 offenbart wirkstoffhaltige Pflaster, die einen oder mehrere Wirkstoffe, wie zum Beispiel Scopolamin, in Form von versponnen Fasern oder Fäden in einer auf einem Trägermaterial aufgebrachten Klebemasse enthalten. In der DE 696 22 780 T2 werden spezielle wirkstoffhaltige Pflaster beschrieben, bei denen ein Wirkstoff wie Scopolamin in Mikrokapseln eingeschlossen ist. Beide vorstehend genannten transdermalen therapeutischen Systeme können neben dem Wirkstoff verschiedene Additive enthalten.

Die DE 10 2004 059 674 B4 beschreibt ein transdermales System zur Abgabe von Scopolamin aus dem 500 µg bis 2 mg Scopolamin über einen Zeitraum von mindestens bis zu 72 h abgegeben werden sollen, wobei dieses System keine die Geschwindigkeit der Freisetzung kontrollierende Membran enthält. Das der Offenbarung der DE 10 2004 059 674 B4 zugrundeliegende Matrixpolymer beruht auf Styrol-Butadien-Copolymerhaftklebstoffen, insbesondere auf einem Styrol-Butadien-Styrol-Haftklebstoff. Für das letztgenannte System wurde allerdings im Vergleich zum Referenzsystem Transderm Scop^{®} eine merklich erhöhte Freisetzung an Scopolamin aus dem TTS beobachtet. Die EP 0 356 382 A2 beschreibt ein transdermales System zur Abgabe von Scopolamin, wobei der Wirkstoff in einer Reservoir- und einer haftenden Hautkontaktschicht vorhanden ist.

Es besteht gegenüber den auf dem Markt vertretenen Produkten ein Bedarf nach einer transdermalen Applikationsform für Scopolamin, die relativ einfach aufgebaut ist und bei der auf eine die Freisetzung von Scopolamin kontrollierende Membran verzichtet werden kann. Darüber hinaus besteht ein Bedarf an einem Verabreichungssystem für Scopolamin, das in der Anfangsphase nach dem Befestigen des Abgabesystems auf der Haut einen hohen Anfangsflux vermittelt, damit relativ schnell ein therapeutisch wirksames Level an Wirkstoff im Körper aufgebaut werden kann. Andererseits sollte das transdermale Applikationssystem eine konstante Wirkstoffzufuhr und einen konstanten wirksamen Level an Wirkstoff über die Tragdauer (zum Beispiel drei Tage) sicherstellen.

Weiterhin besteht ein Bedarf nach einem Verabreichungssystem bei dem die Gefahr einer Kreuzkontamination mit Scopolamin reduziert ist. Die vorliegende Erfindung befasst sich mit diesen Bedarfen.

### Detaillierte Beschreibung

Gemäß einem ersten Aspekt betrifft die vorliegende Erfindung ein transdermales therapeutisches System ohne freisetzungsbestimmende Membran zur Abgabe von Scopolamin, umfassend eine Hautkontaktschicht, eine wirkstoffhaltige Reservoirschicht, eine wirkstoffundurchlässige Rückschicht, sowie gegebenenfalls eine von der Hautkontaktschicht abziehbare Schutzschicht, wobei die Hautkontaktschicht in direktem Kontakt zur wirkstoffhaltigen Reservoirschicht steht, wobei der Wirkstoff in Form der freien Base von Scopolamin vorliegt, der Wirkstoff in der wirkstoffhaltigen Hautkontaktschicht gelöst und in der wirkstoffhaltigen Reservoirschicht teilweise suspendiert vorliegt.

Als Wirkstoff enthält das transdermale therapeutische System Scopolamin als freie Base, wobei geringe Anteile (d.h. 10 Gew.-% oder weniger) an Scopolaminsalzen, wie zum Beispiel das Hydrohalogenid und insbesondere Hydrobromid, nicht schädlich sind. Im Kontext der vorliegenden Erfindung ist die Verwendung ausschließlich der freien Base von Scopolamin als Wirkstoff.

Damit in der Zeit nach dem Aufbringen des TTS ein hoher Anfangsflux erzielt wird, weist die Hautkontaktschicht zweckmäßig eine hohe thermodynamische Aktivität auf. Dies wird zweckmäßig erreicht, indem die Hautkontaktschicht Scopolamin als gelösten Wirkstoff enthält, wobei die thermodynamische Aktivität des Wirkstoffes dadurch hochgehalten wird, indem der Permeationsverstärker/Lösungsvermittler schneller in die Haut diffundiert als der Wirkstoff. Der langsamer als der Permeationsverstärker/Lösungsvermittler diffundierende Wirkstoff bleibt in Lösung und weist durch den reduzierten Gehalt an Permeationsverstärker/Lösungsvermittler eine hohe thermodynamische Aktivität auf, sodass in kurzer Zeit (d.h. etwa 16 Stunden nach dem Aufbringen) ein hoher Anfangsflux generiert wird. Als geeigneter Wirkstoffgehalt für die Hautkontaktschicht kann ein Wirkstoffgehalt im Bereich von 1,2 bis 13 Gew.-% und bevorzugt 9 bis 11 Gew.-% angegeben werden. Für eine geeignete Menge an Wirkstoff in der Hautkontaktschicht kann ein Bereich von 0,06 bis 0,5 mg und bevorzugt 0,1 bis 0,4 mg angegeben werden.

Der wesentliche Anteil des Wirkstoffs Scopolamin befindet sich im erfindungsgemäßen transdermalen therapeutischen System in der Reservoirschicht. Diese enthält demzufolge eine im Vergleich zur Hautkontaktschicht signifikant größere Wirkstoffmenge, wobei eine Wirkstoffmenge im Bereich von 1 bis 1,44 mg und bevorzugt 1,04 bis 1,3 mg als besonders günstig angegeben werden kann. Der Wirkstoffgehalt in der Reservoirschicht beträgt zweckmäßig 7 bis 20 Gew.-% und bevorzugt 8 bis 11 Gew.-%.

Der Wirkstoff in der wirkstoffhaltigen Reservoirschicht liegt teilweise suspendiert vor. Ist der Wirkstoff teilweise suspendiert, zeichnet er sich zweckmäßig durch eine günstige Partikelgröße aus, die definiert wird durch eine D10 von etwa 5 µm, eine D90 von etwa 15 µm und eine D99 von etwa 35 µm. Die Angabe "etwa" bezeichnet in diesem Zusammenhang insbesondere eine Abweichung von dem genannten Wert um ±50%, bevorzugt um ±30%, weiter bevorzugt um ±20% und noch weiter bevorzugt um ±10%. Durch diese Partikelgröße wird die kontinuierliche Abgabe einer therapeutisch wirksamen Menge an Wirkstoff über eine die gesamte Nutzungszeit des transdermalen therapeutischen Systems begünstigt, ohne dass es hierzu einer freisetzungsbestimmenden Membran bedarf. Im Kontext dieser Anmeldung sind die D10, D90 und D99 mit Hilfe von Laserbeugung zu bestimmen, wie dies in European Pharmacopoeia 8.0 (2013), Kapitel 2.9.31 beschrieben ist. In der Angabe D(Zahlenwert) steht der Zahlenwert für den Anteil der Partikel (in Prozent), die kleiner oder gleich groß sind wie als die angegebene Partikelgröße (d.h. bei einer D90 von 15 µm haben 90% der Partikel eine Größe von ≤ 15 µm).

Damit das transdermale therapeutische System eine therapeutisch wirksame Menge an Scopolamin über einen Zeitraum von 72 h bereitstellen kann, ist es weiterhin zweckmäßig, wenn es eine Gesamtmenge von etwa 0,8 bis 2,0 mg, bevorzugt 1,2 bis 1,5 mg Scopolamin und besonders bevorzugt 1,35 bis 1,45 mg Scopolamin enthält.

Als Matrixpolymer für das Scopolamin in der Hautkontaktschicht und in der Reservoirschicht ist ein Polymer geeignet, das eine hohe Diffusibilität aufweist, wie beispielsweise Silikonhaftpolymere.

Die Hautkontaktschicht und die Reservoirschicht beruhen vorzugsweise auf haftvermittelnden Polymeren, die für die Hautkontaktschicht und die Reservoirschicht gleich oder verschieden sein können. Aus Gründen der Kompatibilität ist es aber im Rahmen der vorliegenden Erfindung bevorzugt, wenn die Hautkontaktschicht und die Reservoirschicht auf der selben Sorte von haftvermittelnden Polymeren beruhen, wobei es besonders bevorzugt ist, wenn die Hautkontaktschicht und die Reservoirschicht auf dem gleichen Polymer beruhen, d.h. die Kleberpolymere der Hautkontaktschicht und der Reservoirschicht sind in diesem Fall identisch.

Die Angabe "beruht" bedeutet im vorstehenden Zusammenhang insbesondere, dass der wesentliche Anteil der jeweiligen Schicht auf das bezeichnete haftvermittelnden Polymeren zurückgeht, d.h., dass dieses den größten Massenanteil an der Zusammensetzung der Schicht ausmacht. Dabei kann der Massenanteil ggf. auch 40 Gew.-% oder weniger betragen, wenn alle anderen Bestandteile der Zusammensetzung in Mengen vorliegen, die jeweils geringer sind als 40 Gew.-%. Bevorzugt ist es jedoch, wenn das haftvermittelnde Polymer einen Anteil von mindestens 50 Gew.-%, weiter bevorzugt mindestens 60 Gew-% und noch weiter bevorzugt mindestens 70 Gew-% ausmacht. Ganz besonders bevorzugt ist es, wenn die jeweilige Schicht, die auf einem haftvermittelnden Polymer als Basispolymer beruht, im Wesentlichen (d.h. zu mindestens 90 Gew.-% und insbesondere zu mindestens 99 Gew.-%) und insbesondere ausschließlich dieses Polymer als haftvermittelnden Polymer enthält.

Ein im Rahmen der vorliegenden Erfindung zur Verwendung in der Hautkontaktschicht und der Reservoirschicht besonders geeignetes Polymer ist ein Amin-resistentes Silikonhaftpolymer. Amin-resistente Silikonhaftpolymere zeichnen sich dadurch aus, dass sie keine freien SiOH-Gruppen aufweisen. Entsprechende Silikonhaftpolymere sind aus regulären SiOH-Gruppenenthaltenden Silikonhaftpolymere durch Umwandlung der SiOH- in SiOCH₃-Gruppen erhältlich. Derartige Silikonhaftpolymere sind beispielsweise in der EP-A-0 180 377 beschrieben.

Die genannten Silikonhaftpolymere weisen zudem den Vorteil auf, dass sie in unpolaren Lösungsmitteln, wie z.B. n-Heptan, gut löslich sind, während diese Lösungsmittel über ein nur geringes Lösevermögen für Scopolaminbase verfügen. Es besteht daher bei Verwendung von Silikonhaftpolymeren der vorgenannten Art als Basispolymer die Möglichkeit, den Wirkstoff in eine Lösung der Silikonhaftpolymere einzuarbeiten, ohne dass der Wirkstoff in der Haftpolymerlösung komplett gelöst wird. Das Lösungsmittel wird dazu bei Temperaturen entfernt, die unterhalb der Schmelztemperatur von Scopolaminbase liegen.

Zusätzlich zu Scopolamin kann die Reservoirschicht und/oder die Hautkontaktschicht einen oder mehrere Permeationsverstärker enthalten. Permeationsverstärker reduzieren die Barrierewirkung der menschlichen Haut und erhöhen damit die Permeation des Wirkstoffes in die Haut.

Bevorzugt als Permeationsverstärker werden Fettsäuren, Alkohole oder Ether eingesetzt. Als besonders geeignet haben sich dabei Ölsäure, 2-(2-Ethoxyethoxy-ethanol (Transcutol) und Dipropylenglycol erwiesen, welche in den eingesetzten Konzentrationen nicht zu Hautreizungen führen und mit Silikonhaftpolymeren kompatibel sind. Ein zusätzlicher Vorteil dieser Substanzen ergibt sich daraus, dass sie die sehr geringe Löslichkeit von Scopolaminbase in den Silikonhaftpolymeren erhöhen.

Als geeigneter Anteil für Permeationsverstärker kann, soweit ein solcher vorhanden ist, ein Bereich von 3 bis 12 Gew.-% und insbesondere 6 bis 10 Gew.-% angegeben werden.

Weiterhin kann die Reservoirschicht und/oder die Hautkontaktschicht Löslichkeitsvermittler enthalten, die die Löslichkeit für Scopolamin in der Reservoirsschicht und/oder in der Hautkontaktschicht erhöhen. Durch den Zusatz von Löslichkeitsvermittlern kann eine relativ hohe gelöste Wirkstoffmenge auf einer relativ kleinen Fläche (z.B. etwa 1 cm²) verfügbar gemacht werden.

Geeignete Löslichkeitsvermittler sind beispielsweise Tenside. So kann durch Zugabe von geringen Mengen eines Tensids sichergestellt werden, dass sich schon nach kurzer Zeit ein therapeutisch wirksamer Level an Wirkstoff im Blut des Trägers einstellt. Ein im Rahmen der vorliegenden Erfindung besonders geeignetes Tensid ist beispielsweise Polyoxyethylen (4) laurylether, der kommerziell z.B. als BrijL4 erhältlich ist.

In Bezug auf die vorgenannten Permeationsverstärker und Löslichkeitsvermittler ist anzumerken, das Verbindungen sowohl als Permeationsverstärker als auch als Löslichkeitsvermittler wirksam sein können. In diesem Fall enthält die Reservoirschicht und/oder die Hautkontaktschicht Permeationsverstärker und Löslichkeitsvermittler in Form von einer Verbindung.

Sofern vorhanden, werden der oder die Löslichkeitsvermittler zweckmäßig mit einem Gehalt im Bereich von 0,5 bis 5 Gew.-% und bevorzugt 1 bis 3 Gew.-% in die Reservoirschicht und/oder die Hautkontaktschicht einbezogen.

Um Permeationsverstärker und/oder Löslichkeitsvermittler gleichmäßig in der Klebermatrix zu verteilen, können diese mit einem Verdicker angedickt werden. Geeignet als Verdicker sind beispielsweise Cellulosederivate wie Ethylcellulose. Durch den Zusatz von Verdickern lässt sich die Stabilität des transdermalen therapeutischen Systems vorteilhaft beeinflussen. Sofern vorhanden, werden der oder die Verdicker zweckmäßig mit einem Gehalt im Bereich von 0,1 bis 2 Gew.-% und bevorzugt 0,5 bis 1,2 Gew.-% in die Reservoirschicht und/oder die Hautkontaktschicht einbezogen.

Andere Additive, wie z.B. Silikonöle, können verwendet werden, um die physikalischen Eigenschaften der Kleberstriche, beispielsweise deren Klebrigkeit, zu verbessern. Dies kann insbesondere für die Hautkontaktschicht sinnvoll sein, um die Anhaftung des transdermalen therapeutischen Systems auf die Haut zu verbessern. In einer bevorzugten Ausführungsform enthält die Hautkontaktschicht des hier beschriebenen transdermalen therapeutischen Systems daher einen Klebrigkeitsverbesserer, bevorzugt in Form eines Silikonöls.

Sofern vorhanden, werden der oder die Klebrigkeitsverbesserer zweckmäßig mit einem Gehalt im Bereich von 3 bis 15 Gew.-% und bevorzugt 8 bis 12 Gew.-% in die Reservoirschicht und/oder die Hautkontaktschicht einbezogen.

Wie sich aus dem vorstehenden ergibt gibt es bei den Bestandteilen der Hautkontaktschicht und der Reservoirschicht Überschneidungen. In der Regel sind die Zusammensetzungen der Hautkontaktschicht und der Reservoirschicht aber nicht identisch, d.h. es bestehen Unterschiede in den Bestandteilen der Zusammensetzung oder in deren Anteil in der Zusammensetzung wie zum Beispiel einem höheren Scopolaminanteil in der Reservoirschicht.

Das transdermale therapeutische System gemäß der vorliegenden Erfindung ist bevorzugt so ausgelegt, dass nach Applikation auf die menschliche Haut in 72h ca. 1 mg Wirkstoff aus dem transdermalen therapeutischen System freigesetzt wird.

Alternativ oder zusätzlich dazu ist das transdermale therapeutische System gemäß der vorliegenden Erfindung so ausgelegt, dass der Wirkstoff im Zeitraum von 20 h bis 72 h mit einer Freisetzungsrate von 1,5 bis 15 µg/cm²/h und bevorzugt 3 bis 10 µg/cm²/h aus dem transdermalen therapeutischen System freigesetzt wird. Die Flächenangabe "cm²" bezeichnet dabei die Oberfläche, über die die Hautkontaktschicht mit der Haut in Kontakt steht.

Ganz besonders bevorzugt ist es, wenn das erfindungsgemäße transdermale therapeutische System "bioäqivalent" zu dem bereits zugelassenen Transderm Scop^{®} ist, da in diesem Fall kein gesondertes aufwändiges Zulassungsverfahren durchlaufen werden muss. Als "bioäqivalent" werden nach der WHO zwei pharmazeutische Produkte bezeichnet, wenn sie pharmazeutisch äquivalent oder pharmazeutische Alternativen sind, und ihre Bioverfügbarkeiten, ausgedrückt in Rate (Cmax und tmax) und dem Ausmaß der Absorption (Bereich unter der Kurve) nach der Verabreichung der gleichen molaren Dosis unter den gleichen Bedingungen zu einem solchen Grad ähnlich sind, das angenommen werden kann, dass ihre Wirkungen im Wesentlichen gleich sind (sh. WHO Guidance for organizations performing in vivo bioequivalence studies. WHO Technical Report Series No. 996, 2016, Annex 9). Dabei bezeichnet Cmax die maximale Plasmakonzentration und tmax die Zeit, bis diese erreicht wird. Ein Arzneimittel wird im Rahmen der vorliegenden Erfindung als "bioäqivalent" zu Transderm Scop^{®} eingestuft, wenn es innerhalb eines 90% Konfidenzintervalls eine Bioverfügbarkeit von 80 bis 125% des Referenzarzneimittels bereitstellt.

Ein ganz besonders bevorzugtes transdermales therapeutisches System gemäß der Erfindung gibt im Zeitraum von der Applikation auf die Haut bis zum Zeitpunkt von 72 h nach der Applikation etwa 1 mg Scopolamin (d.h. ± 10%) mit etwa konstanter Rate (d.h. Variation der Rate von höchstens ± 20% und bevorzugt höchstens ± 10%) ab.

Die Auflagefläche der Hautkontaktschicht des trandermalen therpeutischen Systems orientiert sich an der spezifischen Wirkstofffreisetzungsrate und der wirksamen Dosis des Wirkstoffs, liegt jedoch meist im Bereich von 0,5 bis 3 cm², insbesondere 0,8 bis 2 cm², weiter bevorzugt 1 bis 2 cm² und noch weiter bevorzugt 1 bis 1,8 cm².

Die Form der Auflagefläche unterliegt keinen relevanten Beschränkungen, so dass eckige, wie insbesondere quadratische oder rechteckige Formen, aber auch runde Formen, wie zum Beispiel kreisförmige oder ovale Formen, realisiert werden können. Im Rahmen der vorliegenden Erfindung sind runde Ausführungsformen und insbesondere kreisförmige Ausführungsformen bevorzugt.

Um ein Anhaften des transdermalen therapeutischen Systems über die gesamte Tragedauer von 72h zu begünstigen und einer Kreuzkontamination (z.B. der Augen, was bereits bei kleinen Kontaktmengen von Scopolamin zu Pupillenerweiterung und vorübergehenden Sehbeeinträchtigungen führt) vorzubeugen, kann das System mit einem wirkstofffreien Überpflaster ausgestattet sein. Das Überpflaster weist eine im Vergleich zu Auflagefläche der Hautkontaktschicht auf der Haut größere Oberfläche auf, so dass das Überpflaster über den gesamten Umfang der Hautkontaktschicht über diese hinausragt. Wird ein so ausgestattetes transdermales therapeutisches System auf der Haut befestigt, entsteht über den gesamten Umfang der Hautkontaktschicht ein Bereich, in dem das Überpflaster direkt auf der Haut haftet.

Das erfindungsgemäße transdermale therapeutische System ist zur Verwendung in der Behandlung von Bewegungskrankheit und/oder postoperativer Übelkeit geeignet.

Ein weiterer Aspekt der vorliegenden Erfindung betrifft schließlich ein Verfahren zur Herstellung eines Scopolamin-haltigen Pflasters, wie vorstehend angegeben, umfassend die Schritte:
a) Aufbringen einer Scopolamin-haltigen Reservoirschicht umfassend Scopolamin, ein Haftpolymer und gegebenenfalls Permeationsverstärker auf eine wirkstoffundurchlässige Rückschicht, wobei das Scopolamin in der Reservoirschicht teilweise in Form von nicht gelösten Partikeln vorliegt,
b) Aufbringen einer Scopolamin-haltigen Hautkontaktschicht umfassend Scopolamin, ein Haftpolymer und gegebenenfalls Permeationsverstärker sowie klebkraftverstärkenden Substanzen, wobei das Scopolamin in der Hautkontaktschicht in gelöster Form vorliegt, auf eine abziehbare Schutzschicht,
c) Laminieren der Hautkontaktschicht aus b) auf die Reservoirschicht aus a).

Das in c) hergestellte Laminat kann anschließend mit einem Überpflaster, wie vorstehend beschrieben, modifiziert werden, indem das Überpflaster mit der wirkstoffundurchlässigen Rückschicht des in c) hergestellten Laminats verbunden wird.

Im Folgenden wird die Erfindung anhand von Beispielen näher illustriert, die jedoch keine Auswirkungen auf die Interpretation des Schutzumfangs der vorliegenden Erfindung haben sollen.

### Beispiel 1

### Herstellung der Reservoirschicht

Für die Herstellung der Reservoirschicht wurde 13,78% Scopolaminbase, 3% Ölsäure und 83,22% Bio-PSA 4301 (60% Polymerfestgehalt in Heptan) als Suspension formuliert. Die Suspension wurde mit einer Rakel auf eine Fluoropolymer beschichtete Polyesterfolie aufgebracht und für 30 min bei 40°C getrocknet. Das Beschichtungsgewicht des getrockneten Films wird auf 90 g/m² eingestellt. Der getrocknete Film wurde anschließend mit einer 19 µm dicken Polyesterfolie abgedeckt.

### Herstellung der Hautkontaktschicht

Zur Herstellung der Hautkontaktschicht wurden 3% Scopolaminbase, 3% Ölsäure und 94% Bio-PSA 4301 als Lösung formuliert. Die Lösung wurde mit einer Rakel auf eine Fluoropolymer beschichtete Polyesterfolie beschichtet und 15 min bei 40°C getrocknet. Das Beschichtungsgewicht des getrockneten Films wird mit 40 g/m² eingestellt. Der getrocknete Film wurde anschließend mit dem Laminat aus Reservoirsschicht/Polyesterfolie, von dem zuvor die Fluoropolymer beschichtete Folie abgezogen wurde, kaschiert.

Es resultiert ein wirkstoffhaltiges Laminat bestehend aus 19µm Polyesterabdeckfolie, Reservoirschicht, Hautkontaktschicht und Fluoropolymer beschichtete Polyesterfolie.

Aus dem Gesamtlaminat wurden 1 cm² große Pflaster ausgestanzt.

### Beispiel 2

Die Herstellung erfolgte analog Beispiel 1, wobei die Hautkontaktschicht mit einem Anteil von 1,2% Scopolamin, 3% Ölsäure und 95,8% Bio-PSA 4301 und die Reservoirschicht mit einem Anteil von 16,25% Scopolamin, 3% Ölsäure und 80,75% Bio-PSA 4301 formuliert wurde. Die Hautkontaktschicht wurde mit einem Flächengewicht von 50 g/m² und die Reservoirschicht mit einem Flächengewicht von 80 g/m² hergestellt.

### Beispiel 3 (Referenzbeispiel)

### Herstellung der Reservoirschicht

Für die Herstellung der Reservoirschicht wurde 10,4% Scopolaminbase, 8% Transcutol, 1,1% Ethylcellulose, 2% BrijL4, und 78,5% Bio-PSA 4201 als Dispersion formuliert. Die Dispersion wurde mit einer Rakel auf eine Fluoropolymer beschichtete Polyesterfolie beschichtet und für 11 min bei Raumtemperatur getrocknet. Das Beschichtungsgewicht des getrockneten Films wird auf 100 g/m² eingestellt. Der getrocknete Film wurde anschließend mit einer 19 µm dicken Polyesterfolie abgedeckt.

### Herstellung der Hautkontaktschicht

Zur Herstellung der Hautkontaktschicht wurden 10% Scopolaminbase, 7,7% Transcutol, 1%Ethylcellulose, 2% BrijL4, 10,6% Siliconöl und 68,7% Bio-PSA 4201 als Dispersion formuliert. Die Dispersion wurde mit einer Rakel auf eine Fluoropolymer beschichtete Polyesterfolie beschichtet und 4 min bei Raumtemperatur getrocknet. Das Beschichtungsgewicht des getrockneten Films wurde mit 40 g/m² eingestellt. Der getrocknete Film wurde anschließend mit dem Laminat aus Reservoirschicht/Polyesterfolie, von dem zuvor die flourpolymerisierte Folie abgezogen wurde, kaschiert.

### Beispiel 4 (Referenzbeispiel)

### Herstellung der Reservoirschicht

Für die Herstellung der Reservoirschicht wurde 8% Scopolaminbase, 13% Dipropylenglycol, 0,7% Hydroxypropylcellulose, 2% BrijL4, und 76,2% Bio-PSA 4201 als Dispersion formuliert. Die Dispersion wurde mit einer Rakel auf eine Fluoropolymer beschichtete Polyesterfolie beschichtet und für 14 min bei Raumtemperatur getrocknet. Das Beschichtungsgewicht des getrockneten Films wird auf 130 g/m² eingestellt. Der getrocknete Film wurde anschließend mit einer 19 µm dicken Polyesterfolie abgedeckt.

### Herstellung der Hautkontaktschicht

Zur Herstellung der Hautkontaktschicht wurden 10% Scopolaminbase, 16,3% Dipropylenglycol, 0,7% Hydroxypropylcellulose, 2% BrijL4, 9,5% Silikonöl und 61,6% Bio-PSA 4201 als Dispersion formuliert. Die Dispersion wurde mit einer Rakel auf eine Fluoropolymer beschichtete Polyesterfolie beschichtet und 4 min bei Raumtemperatur getrocknet. Das Beschichtungsgewicht des getrockneten Films wird mit 40 g/m² eingestellt. Der getrocknete Film wurde anschließend mit dem Laminat aus Reservoirschicht/Polyesterfolie, von dem zuvor die Fluoropolymer beschichtete Folie abgezogen wurde, kaschiert.

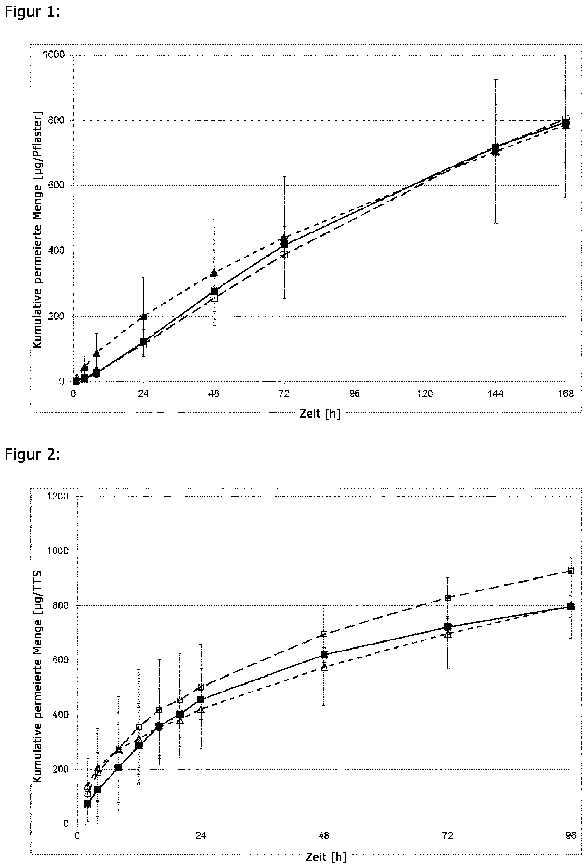

Die Ergebnisse von vergleichenden Permeationsversuchen zwischen einem handelsüblichen Vergleichspräparat (Transderm Scop^{®}) mit den Systemen nach der Erfindung werden in Figur 1 und Figur 2 graphisch dargestellt.

In Figur 1 sind die Permeationsprofile bestimmt als kumulierte Menge an permeiertem Scopolamin gegen die Zeit der Testsysteme nach Beispiel 1 (■, 1 cm²) und Beispiel 2 (□, 1 cm²) gegenüber dem Referenzsystem Transderm Scop (▲, 2,5cm²) als Mittelwert aus sechs Messungen ± Standardabweichung bei Verwendung von dermatomisierter Meerschweinchenhaut aufgetragen.

In Figur 2 sind die Permeationsprofile bestimmt als kumulierte Menge an permeiertem Scopolamin gegen die Zeit der Testsysteme nach Beispiel 3 (■, 1 cm²) und Beispiel 4 (□, 1 cm²) gegenüber dem Referenzsystem Transderm Scop (▲, 2,5cm²) als Mittelwert aus sechs Messungen ± Standardabweichung bei Verwendung von dermatomisierter Meerschweinchenhaut aufgetragen.

## Patentansprüche

1. Transdermales therapeutisches System ohne freisetzungsbestimmende Membran zur Abgabe von Scopolamin umfassend eine Hautkontaktschicht, eine wirkstoffhaltige Reservoirschicht und eine wirkstoff-undurchlässige Rückschicht, sowie gegebenenfalls eine von der Hautkontaktschicht abziehbare Schutzschicht, wobei die Hautkontaktschicht in direktem Kontakt zur wirkstoffhaltigen Reservoirschicht steht, wobei der Wirkstoff in Form der freien Base von Scopolamin vorliegt, der Wirkstoff in der wirkstoffhaltigen Hautkontaktschicht gelöst und in der wirkstoffhaltigen Reservoirschicht teilweise suspendiert vorliegt.

2. Transdermales therapeutisches System gemäß Anspruch 1, wobei die Reservoirschicht eine größere Wirkstoffmenge enthält als die Hautkontaktschicht.

3. Transdermales therapeutisches System gemäß Anspruch 1 oder 2, **dadurch gekennzeichnet, dass** die Hautkontaktschicht einen Wirkstoffgehalt im Bereich von etwa 1,2 bis 13 Gew.-% und bevorzugt 9 bis 11 % aufweist.

4. Transdermales therapeutisches System gemäß einem der Ansprüche 1 bis 3, **dadurch gekennzeichnet, dass** die Reservoirschicht einen Wirkstoffgehalt im Bereich von etwa 7 bis 20 Gew.-% und bevorzugt 8 bis 11 Gew.-% aufweist.

5. Transdermales therapeutisches System gemäß irgendeinem der vorstehenden Ansprüche, **dadurch gekennzeichnet, dass** es eine Gesamtmenge von etwa 0,8 bis 2 mg und bevorzugt 1,2 bis 1,5 mg Scopolamin enthält.

6. Transdermales therapeutisches System gemäß irgendeinem der vorstehenden Ansprüche, **dadurch gekennzeichnet, dass** die Hautkontaktschicht und die Reservoirschicht auf einem selbstklebenden aminresistenten Silikonhaftpolymer als Basispolymer beruht.

7. Transdermales therapeutisches System gemäß irgendeinem vorstehenden Ansprüche, **dadurch gekennzeichnet, dass** die Reservoirschicht und/oder die Hautkontaktschicht Permeationsverstärker, vorzugsweise in Form von einer oder mehreren aus Fettsäuren, Alkoholen oder Ethern, enthält.

8. Transdermales therapeutisches System gemäß Anspruch 7, **dadurch gekennzeichnet, dass** der Permeationsverstärker Ölsäure, 2-(2-Ethoxyethoxy)ethanol und/oder Dipropylenglycol ist.

9. Transdermales therapeutisches System gemäß irgendeinem vorstehenden Ansprüche, **dadurch gekennzeichnet, dass** der Wirkstoff im Zeitraum von 20 h bis 72 h mit einer Freisetzungsrate von 1,5 bis 15 µg/cm²/h und bevorzugt 3 bis 10 µg/cm²/h aus dem transdermalen therapeutischen System freigesetzt wird.

10. Transdermales therapeutisches System gemäß irgendeinem der vorstehenden Ansprüche zur Verwendung in der Behandlung von Bewegungskrankheit und/oder postoperativer Übelkeit.

11. Transdermales therapeutisches System gemäß einem der Ansprüche 1-9, **dadurch gekennzeichnet, dass** die Reservoirschicht und/oder die Hautkontaktschicht einen Löslichkeitsvermittler und/oder einen Verdicker und/oder ein die Klebrigkeit verbesserndes Additiv, bevorzugt in Form von Silikonöl, enthält.

12. Verfahren zur Herstellung eines Scopolamin-haltigen transdermalen therapeutischen Systems gemäß einem der Ansprüche 1 bis 9, 11, umfassend die
Schritte:
a) Aufbringen einer Scopolamin-haltigen Reservoirschicht umfassend Scopolamin, ein Haftpolymer und gegebenenfalls Permeationsverstärker auf eine wirkstoffundurchlässige Rückschicht, wobei das Scopolamin in der Reservoirschicht teilweise in Form von nicht gelösten Partikeln vorliegt,
b) Aufbringen einer Scopolamin-haltigen Hautkontaktschicht umfassend Scopolamin, ein Haftpolymer und gegebenenfalls Permeationsverstärker sowie klebkraftverstärkenden Substanzen, wobei das Scopolamin in der Hautkontaktschicht in gelöster Form vorliegt, auf eine abziehbare Schutzschicht,
c) Laminieren der Hautkontaktschicht aus b) auf die Reservoirschicht aus a).

## Claims

1. Transdermal therapeutic system without release-determining membrane for delivering scopolamine, comprising a skin contact layer, an active substance-containing reservoir layer, an active substance-impermeable backing layer, and optionally a protective layer which is detachable from the skin contact layer, wherein the skin contact layer is in direct contact with the active substance-containing reservoir layer, wherein the active substance is present in the form of the free base of scopolamine, the active substance is present in dissolved form in the active substance-containing skin contact layer and is present in part in suspended form in the active substance-containing reservoir layer.

2. Transdermal therapeutic system according to claims 1, **characterised in that** the reservoir layer contains a greater amount of active substance than the skin contact layer.

3. Transdermal therapeutic system according to claims 1 or 2, **characterised in that** the skin contact layer has an active substance content in the range of from approximately 1.2 to 13 wt.%, and preferably 9 to 11%.

4. Transdermal therapeutic system according to claims 1 to 3, **characterised in that** the reservoir layer has an active substance content in the range of from approximately 7 to 20 wt.%, and preferably 8 to 11 wt.%.

5. Transdermal therapeutic system according to any one of the preceding claims, **characterised in that** it contains a total quantity of scopolamine of from approximately 0.8 to 2 mg, and preferably 1.2 to 1.5 mg.

6. Transdermal therapeutic system according to any one of the preceding claims, **characterised in that** the skin contact layer and the reservoir layer are based on a self-adhesive, amine-resistant silicone adhesive polymer as base polymer.

7. Transdermal therapeutic system according to any one of the preceding claims, **characterised in that** the reservoir layer and/or the skin contact layer contains permeation enhancers, preferably in the form of one or more from fatty acids, alcohols or ethers.

8. Transdermal therapeutic system according to claim 7, **characterised in that** the permeation enhancer is oleic acid, 2-(2-ethoxyethoxy)ethanol and/or is dipropylene glycol.

9. Transdermal therapeutic system according to any one of the preceding claims, **characterised in that** the active substance is released from the transdermal therapeutic system over a period of time from 20 h to 72 h at a release rate of from 1.5 to 15 µg/cm²/h and preferably 3 to 10 µg/cm²/h.

10. Transdermal therapeutic system according to any one of the preceding claims for use in the treatment of motion sickness and/or post-operative nausea.

11. Transdermal therapeutic system according to one of the claims 1 to 9, **characterised in that** the reservoir layer and/or the skin contact layer contains a solubility enhancer and/or a thickening agent and/or a tack-improving additive, preferably in the form of silicone oil.

12. Method for producing a scopolamine-containing transdermal therapeutic system according to one of claims 1 to 9, 11, comprising the steps of:
a) applying, to an active substance-impermeable backing layer, a scopolamine-containing reservoir layer comprising scopolamine, an adhesive polymer and optionally permeation enhancers, wherein the scopolamine is present in the reservoir layer in part in the form of non-dissolved particles,
b) applying, to a detachable protective layer, a scopolamine-containing skin contact layer comprising scopolamine, an adhesive polymer and optionally permeation enhancers as well as adhesive force-enhancing substances, wherein the scopolamine is present in dissolved form in the skin contact layer,
c) laminating the skin contact layer from b) onto the reservoir layer from a).

## Revendications

1. Système thérapeutique transdermique sans membrane régulatrice de libération, destiné à administrer de la scopolamine, comprenant une couche de contact avec la peau, une couche réservoir contenant le principe actif et une couche arrière imperméable au principe actif, ainsi qu'éventuellement une couche de protection pouvant être retirée de la couche de contact avec la peau, dans lequel la couche de contact avec la peau est en contact direct avec la couche réservoir contenant le principe actif, dans lequel le principe actif est présent sous la forme de la base libre de scopolamine, le principe actif est dissous dans la couche de contact avec la peau contenant le principe actif et est présent en partie sous une forme en suspension dans la couche réservoir contenant le principe actif.

2. Système thérapeutique transdermique selon la revendication 1, dans lequel la couche réservoir contient une quantité de principe actif plus importante que la couche de contact avec la peau.

3. Système thérapeutique transdermique selon la revendication 1 ou 2, **caractérisé en ce que** la couche de contact avec la peau présente une teneur en principe actif dans la plage d'environ 1,2 à 13 % en poids et de manière préférée de 9 à 11 %.

4. Système thérapeutique transdermique selon l'une quelconque des revendications 1 à 3, **caractérisé en ce que** la couche réservoir présente une teneur en principe actif dans la plage d'environ 7 à 20 % en poids, et de manière préférée de 8 à 11 % en poids.

5. Système thérapeutique transdermique selon l'une quelconque des revendications précédentes, **caractérisé en ce qu'**il contient une quantité totale d'environ 0,8 à 2 mg et de manière préférée de 1,2 à 1,5 mg de scopolamine.

6. Système thérapeutique transdermique selon l'une quelconque des revendications précédentes, **caractérisé en ce que** la couche de contact avec la peau et la couche réservoir reposent sur un polymère adhésif à base de silicone autocollant résistant à l'amine en tant que polymère de base.

7. Système thérapeutique transdermique selon l'une quelconque des revendications précédentes, **caractérisé en ce que** la couche réservoir et/ou la couche de contact avec la peau contiennent des activateurs de perméation, de préférence sous la forme d'un ou de plusieurs parmi des acides gras, des alcools ou des éthers.

8. Système thérapeutique transdermique selon la revendication 7, **caractérisé en ce que** l'activateur de perméation est un acide gras, du 2-(2-éthoxyéthoxy)éthanol et/ou du dipropylèneglycol.

9. Système thérapeutique transdermique selon l'une quelconque des revendications précédentes, **caractérisé en ce que** le principe actif est libéré du système thérapeutique transdermique dans la période de temps de 20 h à 72 h à une vitesse de libération de 1,5 à 15 µg/cm²/h et de manière préférée de 3 à 10 µg/cm²/h.

10. Système thérapeutique transdermique selon l'une quelconque des revendications précédentes destiné à être utilisé lors du traitement d'une cinétose et/ou de nausées postopératoires.

11. Système thérapeutique transdermique selon l'une quelconque des revendications 1 - 9,
**caractérisé en ce que** la couche réservoir et/ou la couche de contact avec la peau contiennent un agent solubilisant et/ou un agent épaississant et/ou un additif améliorant l'adhérence, de préférence sous la forme d'une huile de silicone.

12. Procédé de fabrication d'un système thérapeutique transdermique contenant de la scopolamine selon l'une quelconque des revendications 1 à 9, 11, comprenant les étapes :
a) d'application d'une couche réservoir contenant de la scopolamine comprenant de la scopolamine, un polymère adhésif et éventuellement des activateurs de perméation sur une couche arrière imperméable au principe actif, dans lequel la scopolamine est présente dans la couche réservoir en partie sous la forme de particules non dissoutes,
b) d'application d'une couche de contact avec la peau contenant de la scopolamine, comprenant de la scopolamine, un polymère adhésif et éventuellement des activateurs de perméation ainsi que des substances renforçant la force d'adhérence, dans lequel la scopolamine est présente dans la couche de contact avec la peau sous une forme dissoute, sur une couche de protection pouvant être retirée,
c) de stratification de la couche de contact avec la peau issue de b) sur la couche réservoir issue de a).
